# EUROPÄISCHE PATENTSCHRIFT

(11) **EP 0 905 123 B1**
(45) Veröffentlichungstag und Bekanntmachung des Hinweises auf die Patenterteilung: **12.12.2001**
(21) Anmeldenummer: 98117339.6
(22) Anmeldetag: 12.09.1998
(51) Int. Cl.: C07C 217/90, C07D 213/69

(54) **o-Amino(thio)phenol-carbonsäuren und deren Herstellung**
o-amino(thio)phenol-carboxylic acids and their preparation
Acides o-amino(thio)phenol-carboxyliques et leur préparation

(30) Priorität: 24.09.1997 DE 19742194
(43) Veröffentlichungstag der Anmeldung: 31.03.1999
(73) Patentinhaber: Infineon Technologies AG, 81669 München (DE)
(72) Erfinder: Sezi, Recai, Dr., 91341 Röttenbach (DE); Keitmann, Michael, 91074 Herzogenaurach (DE)
(74) Vertreter: Müller . Hoffmann & Partner Patentanwälte

(56) Entgegenhaltungen:
- EP-A- 0 023 662
- EP-A- 0 264 678

## Beschreibung

Die Erfindung betrifft neue o-Aminophenol-carbonsäuren und o-Aminothiophenol-carbonsäuren, die zusammen kurz auch als o-Amino(thio)phenol-carbonsäuren bezeichnet werden, sowie ein Verfahren zu deren Herstellung.

o-Aminophenol-carbonsäuren werden insbesondere zur Herstellung von hochtemperaturstabilen Polymeren, wie Polybenzoxazolen (PBO) bzw. deren Vorstufen, benötigt. Im Vergleich zur Herstellung der Polybenzoxazole bzw. der PBO-Vorstufen aus Bis-o-aminophenolen und Dicarbonsäuren hat die Verwendung von o-Aminophenol-carbonsäuren deutliche Vorteile. So kann eine o-Aminophenol-carbonsäure mit sich selbst umgesetzt werden, d.h. für die Polymerisation ist ein zweites Monomer nicht unbedingt erforderlich. Hierdurch können die Reinheitskontrolle und die Lagerhaltung vereinfacht werden. Außerdem ist die Stöchiometrie bereits vorgegeben, d.h. Fehler bei der Berechnung oder der Einwaage der Reaktanten, wie sie bei der Reaktion von Bis-o-aminophenolen mit Dicarbonsäuren auftreten können, sind bei der Verwendung von o-Aminophenol-carbonsäuren ausgeschlossen. Ferner beeinflußt die Art des verwendeten Monomers in starkem Maße das Eigenschaftsspektrum der damit hergestellten PBO-Vorstufe bzw. des Polybenzoxazols. So werden nicht nur das thermische, elektrische und mechanische Verhalten, sondern auch die Löslichkeit und die Hydrolysestabilität sowie zahlreiche weitere Eigenschaften des Polymers durch das bei der Herstellung verwendete Monomer stark beeinflußt.

PBO-Vorstufen können in Form einer photosensitiven Zusammensetzung kostengünstig auf direktem Weg, d.h. ohne einen Hilfsresist, strukturiert werden. Im Vergleich mit anderen direkt photostrukturierbaren Dielektrika, wie Polyimid (PI) und Benzocyclobuten (BCB), bieten PBO-Vorstufen den Vorteil der positiven Strukturierbarkeit und der wäßrig-alkalischen Entwicklung (siehe EP 0 023 662 B1 und EP 0 264 678 B1). Hierzu müssen die verwendeten PBO-Vorstufen bei der Belichtungswellenlänge weitgehend transparent und im - vorzugsweise metallionenfreien - Entwickler ausreichend löslich sein. Wie die Polyimide haben auch Polybenzoxazole den großen Vorteil, daß sie - im Vergleich zum cyclisierten Endprodukt - als gut lösliche Vorstufe auf ein Substrat aufgebracht und anschließend cyclisiert werden können, wobei die Löslichkeit und damit die Sensibilität gegenüber Lösemitteln und anderen Prozeßchemikalien stark abnimmt.

Für den Einsatz von Polybenzoxazolen in der Mikroelektronik ist neben einer guten Löslichkeit der Vorstufen auch eine niedrige Feuchteaufnahme und ein gutes Planarisierungsvermögen von Vorteil. So können bei der Verwendung eines gut planarisierenden Dielektrikums bei der Herstellung von Bauteilen kostenintensive Schleifprozeduren (Chemical Mechanical Polishing; CMP) vermieden werden.

o-Aminophenol-carbonsauren sind beispielsweise aus GB 811 758 und GB 1 283 476 bekannt. Bei PBO-Filmen, die aus den bekannten Monomeren hergestellt werden, beträgt die Wasseraufnahme in kochendem Wasser nach 24 h 0,77 %. Auf das Planarisierungsverhalten der hergestellten Polymere nach der Cyclisierung auf dem Substrat oder auf deren Eignung als Basispolymere für positiv photostrukturierbare Zusammensetzungen gibt es keine Hinweise.

Aufgabe der Erfindung ist es, o-Aminophenol-carbonsäuren und o-Aminothiophenol-carbonsäuren bereitzustellen, die zur Herstellung von Polymeren geeignet sind, welche die stark gestiegenen Anforderungen der Mikroelektronik erfüllen. Die o-Amino(thio)phenol-carbonsäuren sollen insbesondere die Herstellung gut löslicher Polymer-Vorstufen ermöglichen, die nach der Cyclisierung auf einem Substrat Polybenzoxazole bzw. Polybenzothiazole mit geringer Feuchteaufnahme und hohem Planarisierungsgrad ergeben.

Dies wird erfindungsgemäß durch o-Aminophenol-carbonsäuren und o-Aminothiophenol-carbonsäuren folgender Struktur erreicht: 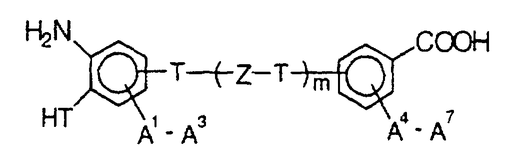 dabei gilt folgendes:
A¹ bis A⁷ sind - unabhängig voneinander - H, CH₃, OCH₃, CH₂CH₃ oder OCH₂CH₃;
T ist O oder S, und m ist 0 oder 1;
Z ist einer der folgenden Reste: 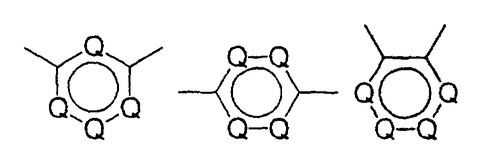 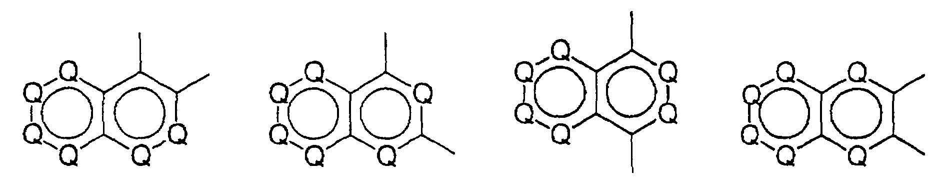 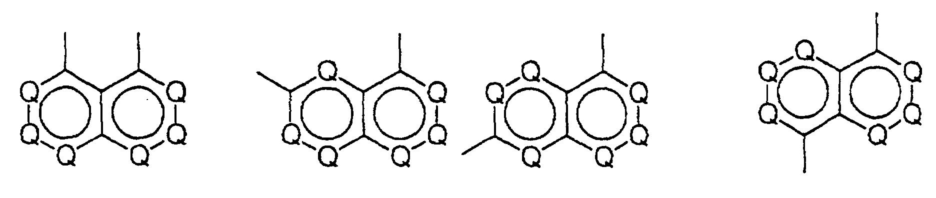 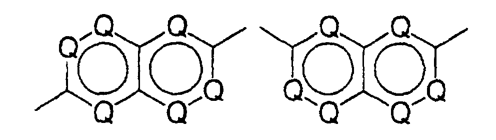 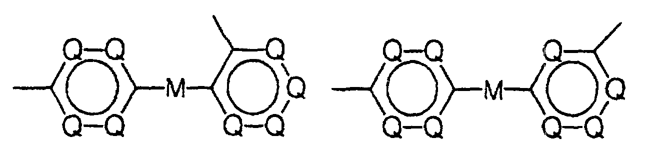 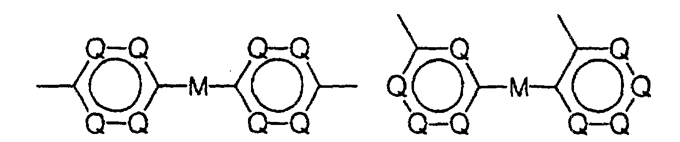 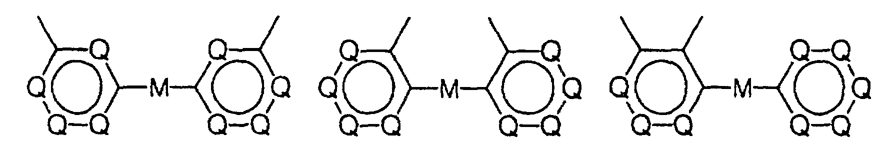 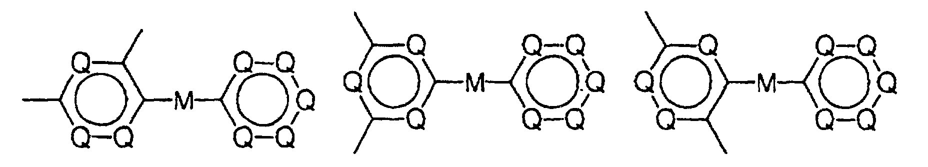 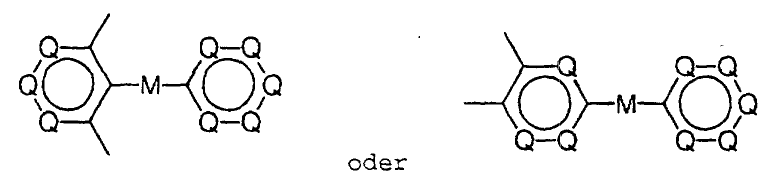
mit
- Q =: C-A oder N,
mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = 0 bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl, wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
- M =: eine Einfachbindung, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃) (C₆H₅), C(CH₃) (C₆F₅), C(CF₃)(C₆H₅), C(CF₃)(C₆F₅), C(C₆H₅)₂, C(C₆F₅)₂, CO, SO₂, 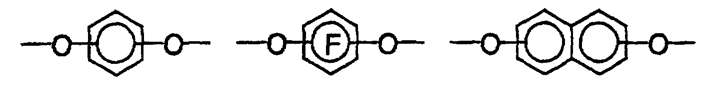 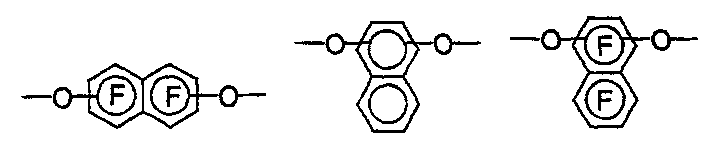 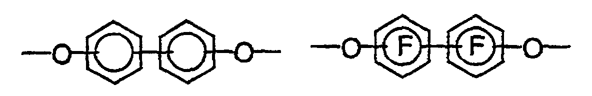 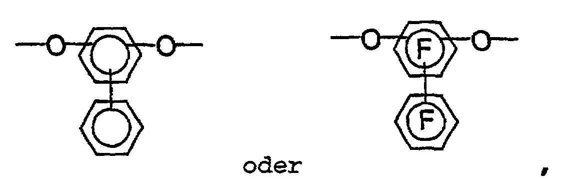
mit der Maßgabe, daß bei m = 0 keine 3-Amino-4-hydroxyphenoxygruppe in p-Stellung zur Carboxylgruppe vorliegen darf.

Die neuen Verbindungen weisen beispielsweise folgende Struktur auf:

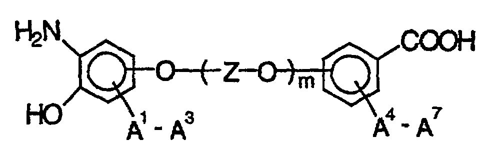

Bei derartigen Verbindungen sind offensichtlich die Etherbrücken für die gute Löslichkeit und die guten Planarisierungseigenschaften der damit hergestellten Polymer-Vorstufen verantwortlich. Im übrigen bedeutet die Charakterisierung "A¹-A³" und "A⁴-A⁷" in der Strukturformel, daß die Aminophenylgruppen Reste A¹, A² und A³ bzw. A⁴, A⁵, A⁶ und A⁷ aufweisen.

Die o-Amino(thio)phenol-carbonsäuren können in der Weise hergestellt werden, daß
(a) eine Halogenverbindung der Struktur 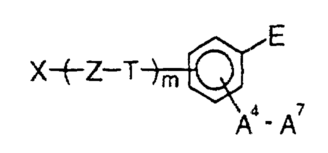 in einem Lösemittel bei einer Temperatur zwischen -10 und 80°C
   mit einem Nitrophenol bzw. Nitrothiophenol (kurz "Nitro(thio)phenol") der Struktur 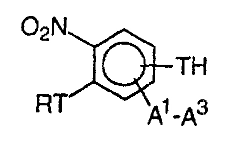 in Gegenwart mindestens der stöchiometrischen Menge einer Base oder mit einem Alkalisalz des Nitro(thio)phenols zur Reaktion gebracht wird,
   wobei X ein Halogenatom ist, E die Bedeutung CN oder COOR¹ mit R¹ = Alkyl (mit 1 bis 5 C-Atomen), Phenyl oder Benzyl hat, A¹ bis A⁷, T und Z wie angegeben definiert sind und R einer der folgenden Reste ist: Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen; und
(b) die dabei gebildete Nitroverbindung zur Aminoverbindung reduziert und hydrolysiert und die Gruppe R abgespalten wird.

Bei diesem Syntheseverfahren, das sehr wirtschaftlich ist, wird somit ein halogenhaltiger Ester oder ein entsprechendes Nitril mit einem Nitro(thio)phenol umgesetzt, das in o-Stellung zur Nitrogruppe eine mit R geschützte Hydroxy- oder Mercaptogruppe aufweist. Die dabei gebildete Nitroverbindung wird dann zur entsprechenden Aminoverbindung reduziert, die Ester- bzw. Nitrilgruppe zur Carboxylgruppe hydrolysiert und die Schutzgruppe R abgespalten.

Die Herstellung von Nitro(thio)phenolen mit einer geschützten Hydroxy- oder Mercaptogruppe in o-Stellung zur Nitrogruppe ist in der gleichzeitig eingereichten deutschen Patentanmeldung Akt.Z. 197 42 135.0 - "o-Nitro(thio)phenolderivate und deren Herstellung" (GR 97 P 3683) beschrieben.

Die Schutzgruppe R ist vorzugsweise eine Alkyl-, Alkoxyalkyl-, Phenyl- oder Benzylgruppe. Dabei ist wichtig, daß der Rest RT bei der Reaktion zwischen der Halogenverbindung und dem Nitro(thio)phenol stabil ist, anschließend aber gespalten werden kann.

Die Reaktion zwischen der Halogenverbindung und dem Nitro(thio)phenol, bei der eine Ether- bzw. Thioetherbrücke gebildet wird, erfolgt in Gegenwart einer Base. Diese Base ist vorzugsweise ein Carbonat oder Hydrogencarbonat eines Alkalioder Erdalkalimetalls, wie Natriumcarbonat und Kaliumcarbonat. Für die (Thio)etherbildung ist mindestens eine stöchiometrische Menge der Base erforderlich. Vorteilhaft kann auch eine organische Base mit einem tertiären N-Atom, beispielsweise Triethylamin und Pyridin, eingesetzt werden.

Anstelle des Nitro(thio)phenols kann auch ein entsprechendes Alkalisalz eingesetzt werden, beispielsweise das Kaliumsalz. In diesem Fall ist für die Umsetzung mit der Halogenverbindung eine Base nicht unbedingt erforderlich.

Als Reaktionstemperatur hat sich der Bereich von -10 bis 80°C als geeignet erwiesen. Temperaturen ≤ 80°C werden wegen der höheren Selektivität der Umsetzung bevorzugt.

Geeignete Lösemittel sind insbesondere Dimethylformamid, Diethylformamid, Dimethylacetamid, Dimethylsulfoxid, N-Methylpyrrolidon, γ-Butyrolacton, Acetonitril, Tetrahydrofuran und Pyridin. Prinzipiell können aber alle polaren aprotischen Lösemittel verwendet werden, in denen die Ausgangsverbindungen löslich sind.

Die Reduktion der Nitroverbindung kann beispielsweise durch Hydrierung mit Wasserstoff an Pd/C durchgeführt werden. Es sind aber prinzipiell alle Verfahren geeignet, die sich für die Reduktion der Nitrogruppe zur Aminogruppe eignen. Die Hydrolyse der Ester- bzw. Nitrilgruppe kann beispielsweise mit Kaliumhydroxid erfolgen. Zur Abspaltung der Schutzgruppe kann beispielsweise Trifluoressigsäure oder Titantetrachlorid eingesetzt werden. Diese Reaktionen können in getrennten Verfahrensschritten erfolgen; die Reihenfolge der Verfahrensschritte ist dabei beliebig.

Es ist auch möglich, die Abspaltung der Schutzgruppe und die Hydrolyse gleichzeitig durchzuführen, d.h. in einem Schritt. Beim Vorliegen einer Estergruppe erfolgen diese beiden Reaktionen besonders vorteilhaft zusammen mit der Reduktion der Nitrogruppe, und zwar vorzugsweise durch eine Hydrierung mit Wasserstoff an Pd/C. Die Hydrierung erfolgt vorzugsweise bei Temperaturen von 25 bis 50°C. Geeignete Lösemittel sind Ester oder Ether, beispielsweise Essigsäureethylester und Tetrahydrofuran.

Alternativ können die o-Amino(thio)phenol-carbonsäuren auch in der Weise hergestellt werden, daß
(a) eine Nitroverbindung der Struktur 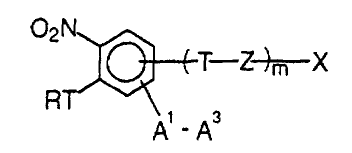 in einem Lösemittel bei einer Temperatur zwischen -10 und 80°C
   mit einem Phenol bzw. Thiophenol (kurz "(Thio)phenol") der Struktur 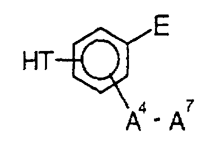 in Gegenwart mindestens der stöchiometrischen Menge einer Base oder mit einem Alkalisalz des (Thio)phenols zur Reaktion gebracht wird,
   wobei X ein Halogenatom ist, E die Bedeutung CN oder COOR¹ mit R¹ = Alkyl (mit 1 bis 5 C-Atomen), Phenyl oder Benzyl hat, A¹ bis A⁷, T und Z wie angegeben definiert sind und R einer der vorstehend angegebenen Reste ist; und
(b) die dabei gebildete Nitroverbindung zur Aminoverbindung reduziert und hydrolysiert und die Gruppe R abgespalten wird.

Bei diesem Herstellungsverfahren, das ebenfalls sehr wirtschaftlich ist, wird somit eine halogenhaltige Nitroverbindung, die in o-Stellung zur Nitrogruppe eine geschützte Hydroxy- oder Mercaptogruppe aufweist, mit einem ester- oder nitrilgruppenhaltigen (Thio)phenol umgesetzt. Die dabei gebildete Nitroverbindung wird dann - in der vorstehend angegebenen Weise - einer Reduktion, Hydrolyse und Spaltung unterworfen.

Die aus den o-Amino(thio)phenol-carbonsäuren nach der Erfindung hergestellten Polymer-Vorstufen, die - im Vergleich zum Stand der Technik - verbesserte Eigenschaften aufweisen, sind in vielen organischen Lösemitteln, wie Aceton, Cyclohexanon, N-Methylpyrrolidon, Diethylenglykolmono- bzw. -diethylether, Ethyllactat und γ-Butyrolacton, sowie in metallionenfreien wäßrig-alkalischen Entwicklern gut löslich. Sie sind deshalb als Basispolymer für positiv photostrukturierbare und wäßrigalkalisch entwickelbare Dielektrika gut geeignet. Die Vorstufen können mittels Schleudertechnik problemlos auf Substrate, wie Siliziumwafer, aufgebracht werden, sie bilden gleichmäßige Filme und lassen sich auf dem Substrat gut cyclisieren. Ein besonderer Vorteil der aus diesen o-Amino(thio)phenol-carbonsäuren hergestellten Vorstufen ist das hohe Planarisierungsvermögen und die geringe Feuchteaufnahme.

Anhand von Ausführungsbeispielen soll die Erfindung noch näher erläutert werden.

### Beispiel 1

### Herstellung von 4-(4-Benzyloxycarbonyl-phenoxy)-nonafluorbiphenyl

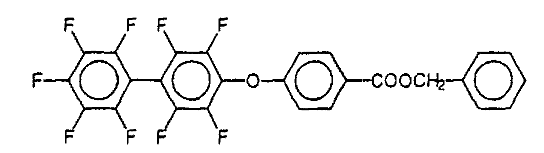

37,4 g Decafluorbiphenyl (0,112 mol) werden in 700 ml Dimethylformamid gelöst und mittels eines Kryostaten auf -10°C abgekühlt, dann wird innerhalb von 2 h eine Lösung von 29,8 g Kalium-4-benzyloxycarbonylphenolat (0,112 mol) in 300 ml Dimethylformamid zugetropft. Nach 48 h bei -10°C ist das Kaliumsalz umgesetzt. Dann wird das Dimethylformamid in einem Rotationsverdampfer entfernt, der Rückstand in wenig Tetrahydrofuran aufgenommen und über eine Kieselgelsäule filtriert. Die erhaltene klare Lösung wird im Rotationsverdampfer eingeengt, bis ein weißer Feststoff ausfällt. Der Feststoff wird dann in n-Hexan gerührt, über ein Faltenfilter abfiltriert und anschließend in einem Vakuumschrank 48 h unter Stickstoff bei 40°C/10 mbar getrocknet (Ausbeute: 92 %).

### Charakterisierung:

- Massenspektrum: Molekülpeak bei 542
- Elementaranalyse:

| | | |
|---|---|---|
| Theoretischer Wert (in %) | C: 57,6 | H: 2,0 |
| Ermittelter Wert (in %) | C: 57,5 | H: 1,9 |

- Schmp: 120°C.

### Beispiel 2

### Herstellung von 4-(4-Nitro-3-benzyloxy-phenoxy)-4'-(4-benzyloxycarbonyl-phenoxy) -octafluorbiphenyl

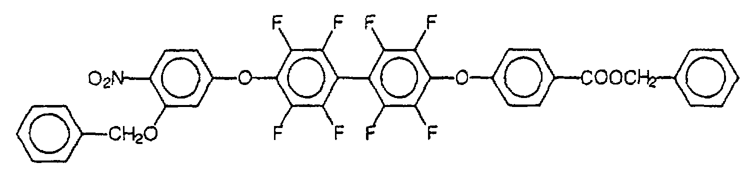

49,9 g des entsprechend Beispiel 1 hergestellten 4-(4-Benzyloxycarbonyl-phenoxy)-nonafluorbiphenyl (0,092 mol) und 26,1 g Kalium-4-nitro-3-benzyloxy-phenolat (0,092 mol) werden in 400 ml Dimethylformamid gelöst, und die Lösung wird auf 80°C erhitzt; nach 24 h ist die Reaktion vollständig abgelaufen. Dann wird das Lösemittel in einem Rotationsverdampfer entfernt. Der erhaltene feste Rückstand wird dreimal mit Methanol gewaschen, über einen Büchnertrichter abfiltriert und anschließend in einem Vakuumschrank 48 h unter Stickstoff bei 40°C/10 mbar getrocknet (Ausbeute: 94 %).

### Charakterisierung:

- Massenspektrum: Molekülpeak bei 767
- Elementaranalyse:

| | | | |
|---|---|---|---|
| Theoretischer Wert (in %) | C: 61,0 | H: 2,8 | N: 1,8 |
| Ermittelter Wert (in %) | C: 60,8 | H: 2,7 | N: 1,9 |

- Schmp: 152°C.

### Beispiel 3

Herstellung von 4-(4-Amino-3-hydroxy-phenoxy)-4'-(4-carboxyphenoxy)-octafluorbiphenyl

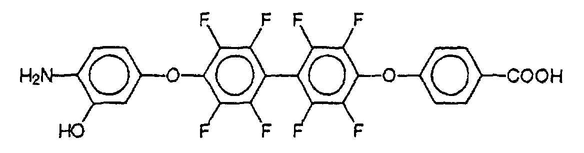

49,9 g des entsprechend Beispiel 2 hergestellten 4-(4-Nitro-3-benzyloxy-phenoxy)-4'-(4-benzyloxycarbonyl-phenoxy)-octafluorbiphenyl (0,065 mol) werden in 400 ml eines Gemisches von Tetrahydrofuran und Essigsäureethylester (Volumenverhältnis 1:1) gelöst, und zu der Lösung werden 5 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die gelbbeige Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 91 %).

### Charakterisierung:

- Massenspektrum: Molekülpeak bei 557
- Elementaranalyse:

| | | | |
|---|---|---|---|
| Theoretischer Wert (in %) | C: 53,9 | H: 2,0 | N: 2,5 |
| Ermittelter Wert (in %) | C: 53,7 | H: 2,1 | N: 2,5 |

- Schmp: 180°C (Zersetzung).

### Beispiel 4

Herstellung von 4-(4-Nitro-3-benzyloxy-phenoxy) -benzoesäurebenzylester 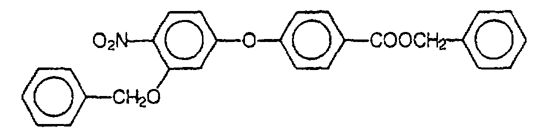 24,7 g 5-Fluor-2-nitrophenyl-benzylether (0,1 mol) werden in 250 ml Dimethylsulfoxid gelöst, dann wird bei Raumtemperatur eine Lösung von 26,6 g des Kaliumsalzes von 4-Hydroxybenzoesäurebenzylester (0,1 mol) in 250 ml Dimethylsulfoxid unter Rühren langsam zugetropft. Danach wird zunächst 1 h bei Raumtemperatur gerührt und dann 24 h bei 50°C. Danach läßt man die Reaktionslösung auf Raumtemperatur abkühlen, filtriert über ein Faltenfilter ab, verdünnt mit 700 ml Wasser und schüttelt das Rohprodukt mit 300 ml Essigsäureethylester aus. Die organische Phase wird dann dreimal mit Wasser gewaschen, über Natriumsulfat getrocknet und in einem Rotationsverdampfer eingeengt, bis das Reaktionsprodukt ausfällt. Das Reaktionsprodukt wird 2 h in Petroleumbenzin (Siedebereich 40 bis 60°C) gerührt, über einen Büchnertrichter abfiltriert und anschließend in einem Vakuumschrank 48 h unter Stickstoff bei 40°C/10 mbar getrocknet (Ausbeute: 91 %).

Charakterisierung:
- Massenspektrum: Molekülpeak bei 455
- Elementaranalyse:

| | | | |
|---|---|---|---|
| Theoretischer Wert (in %) | C: 71,2 | H: 4,6 | N: 3,1 |
| Ermittelter Wert (in %) | C: 71,0 | H: 4,7 | N: 3,0 |

- Schmp: 96°C.

### Beispiel 5

Herstellung von 4-(4-Amino-3-hydroxy-phenoxy)-benzoesäure 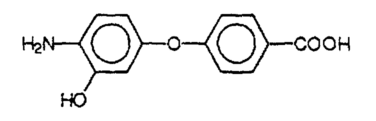 46,6 g des entsprechend Beispiel 4 hergestellten 4-(4-Nitro-3-benzyloxy-phenoxy)-benzoesäurebenzylester (0,11 mol) werden in 500 ml eines Gemisches von Tetrahydrofuran und Essigsäureethylester (Volumenverhältnis 1:1) gelöst, und zu der Lösung werden 5 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die schwach violette Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 93 %).

Charakterisierung:
- Massenspektrum: Molekülpeak bei 245
- Elementaranalyse:

| | | | |
|---|---|---|---|
| Theoretischer Wert(in %) | C: 63,7 | H: 4,5 | N: 5,7 |
| Ermittelter Wert(in %) | C: 63,5 | H: 4,5 | N: 5,8. |

- Schmp: 190°C (Zersetzung)

### Beispiel 6

Herstellung von 2-(4-Benzyloxycarbonyl-phenoxy)-3,4,5,6-tetrafluorpyridin 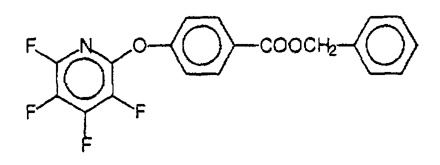 33,8 g Pentafluorpyridin (0,2 mol) werden in 500 ml Dimethylformamid gelöst und mittels eines Kryostaten auf 0°C abgekühlt, dann wird innerhalb von 2 h eine Lösung von 53,3 g Kalium-4-benzyloxycarbonylphenolat (0,2 mol) in 400 ml Dimethylformamid zugetropft. Nach 24 h bei 0°C ist das Kaliumsalz umgesetzt. Dann wird das Dimethylformamid in einem Rotationsverdampfer entfernt, der Rückstand in wenig Tetrahydrofuran aufgenommen und über eine Kieselgelsäule filtriert. Die erhaltene klare Lösung wird im Rotationsverdampfer eingeengt, bis das Reaktionsprodukt ausfällt. Das Reaktionsprodukt wird dann in n-Hexan gerührt, über ein Faltenfilter abfiltriert und anschließend in einem Vakuumschrank 48 h unter Stickstoff bei 40°C/10 mbar getrocknet (Ausbeute: 91 %).

Charakterisierung:
- Massenspektrum: Molekülpeak bei 377
- Elementaranalyse:

| | | | |
|---|---|---|---|
| Theoretischer Wert (in %) | C: 60,5 | H: 2,9 | N: 3,7 |
| Ermittelter Wert (in %) | C: 60,6 | H: 2,9 | N: 3,6 |

### Beispiel 7

Herstellung von 4-(4-Nitro-3-benzyloxy-phenoxy)-2-(4-benzyloxycarbonyl-phenoxy)-3,5,6-trifluorpyridin 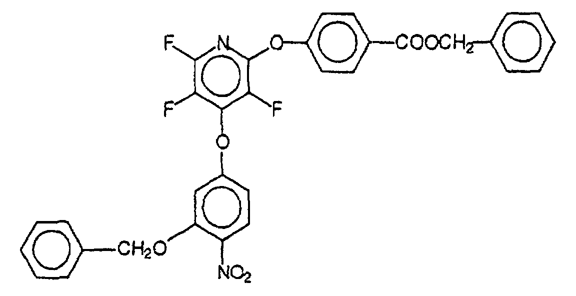 40 g des entsprechend Beispiel 6 hergestellten 2-(4-Benzyloxycarbonyl-phenoxy)-3,4,5,6-tetrafluorpyridin (0,106 mol) und 30 g Kalium-4-nitro-3-benzyloxy-phenolat (0,106 mol) werden in 500 ml Dimethylsulfoxid gelöst. Zur Lösung werden portionsweise 30 g Kaliumcarbonat (0,22 mol) gegeben, dann wird 24 h bei Raumtemperatur gerührt. Anschließend wird 24 h auf 60°C erhitzt, und dann werden 15 g Kaliumhydrogencarbonat (0,15 mol) zugegeben. Danach läßt man die Reaktionslösung auf Raumtemperatur abkühlen und filtriert über ein Faltenfilter ab. Man schüttelt das Rohprodukt mit 300 ml Essigsaureethylester und 700 ml Wasser aus, wäscht die organische Phase dreimal mit Wasser und engt sie in einem Rotationsverdampfer ein, bis das Reaktionsprodukt ausfällt. Das Reaktionsprodukt wird dann in einem Gemisch von Essigsäureethylester und n-Hexan (Volumenverhältnis 1:1) umkristallisiert und anschließend in einem Vakuumschrank 48 h unter Stickstoff bei 40°C/10 mbar getrocknet (Ausbeute: 92 %).

### Charakterisierung:

- Massenspektrum: Molekülpeak bei 602
- Elementaranalyse:

| | | | |
|---|---|---|---|
| Theoretischer Wert(in %) | C: 63,8 | H: 3,5 | N: 4,6 |
| Ermittelter Wert(in %) | C: 63,7 | H: 3,5 | N: 4,6 |

### Beispiel 8

Herstellung von 4-(4-Amino-3-hydroxy-phenoxy)-2-(4-carboxyphenoxy)-3,5,6-trifluorpyridin 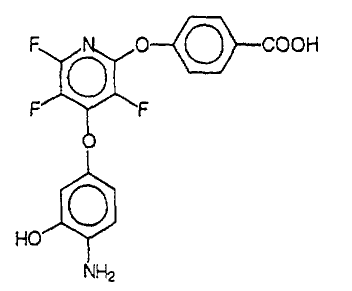 40 g des entsprechend Beispiel 7 hergestellten 4-(4-Nitro-3-benzyloxy-phenoxy)-2-(4-benzyloxycarbonyl-phenoxy)-3,5,6-trifluorpyridin (0,066 mol) werden in 600 ml eines Gemisches von Tetrahydrofuran und Essigsäureethylester (Volumenverhältnis 1:1) gelöst, und zu der Lösung werden 4 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die orangefarbene Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt in einem Vakuumschrank 48 h unter Stickstoff bei 40°C/10 mbar getrocknet (Ausbeute: 91 %).

### Charakterisierung:

- Massenspektrum: Molekülpeak bei 392
- Elementaranalyse:

| | | | |
|---|---|---|---|
| Theoretischer Wert (in %) | C: 55,1 | H: 2,8 | N: 7,1 |
| Ermittelter Wert (in %) | C: 55,1 | H: 2,8 | N: 7,2 |

### Beispiel 9

Herstellung von 2-(4-Benzyloxycarbonyl-phenoxy)-1-trifluormethyl-3,4,5,6-tetrafluorbenzol 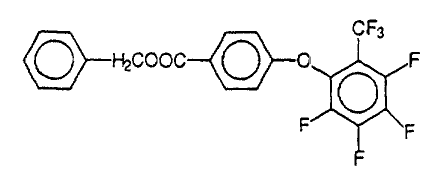 35,4 g Octafluortoluol (0,15 mol) werden in 400 ml Dimethylformamid gelöst und mittels eines Kryostaten auf 0°C abgekühlt, dann wird innerhalb von 2 h eine Lösung von 40 g Kalium-4-benzyloxycarbonylphenolat (0,15 mol) in 300 ml Dimethylformamid zugetropft. Nach 24 h bei 0°C ist das Kaliumsalz umgesetzt. Dann wird das Dimethylformamid in einem Rotationsverdampfer entfernt, der Rückstand in wenig Tetrahydrofuran aufgenommen und über eine Kieselgelsäule filtriert. Die erhaltene klare Lösung wird im Rotationsverdampfer eingeengt, bis das Reaktionsprodukt ausfällt. Das Reaktionsprodukt wird dann in n-Hexan gerührt, über ein Faltenfilter abfiltriert und anschließend in einem Vakuumschrank 48 h unter Stickstoff bei 40°C/10 mbar getrocknet (Ausbeute: 95 %).

### Charakterisierung:

- Massenspektrum: Molekülpeak bei 444
- Elementaranalyse:

| | | | |
|---|---|---|---|
| Theoretischer Wert (in %) | C: 71,2 | H: 4,6 | N: 3,1 |
| Ermittelter Wert (in %) | C: 71,0 | H: 4,7 | N: 3,0 |

### Beispiel 10

Herstellung von 4-(4-Nitro-3-benzyloxy-phenoxy)-2-(4-benzyloxycarbonyl-phenoxy)-1-trifluormethyl-3,5,6-trifluorbenzol 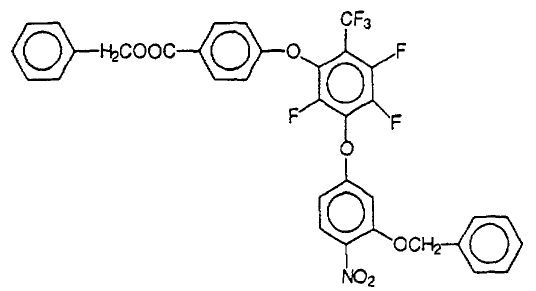 40 g des entsprechend Beispiel 9 hergestellten 2-(4-Benzyloxycarbonyl-phenoxy)-1-trifluormethyl-3,4,5,6-tetrafluorbenzol (0,09 mol) und 25,5 g Kalium-4-nitro-3-benzyloxyphenolat (0,09 mol) werden in 400 ml Dimethylsulfoxid gelöst. Zur Lösung werden portionsweise 30 g Kaliumcarbonat (0,22 mol) gegeben, dann wird 24 h bei Raumtemperatur gerührt. Anschließend wird 24 h auf 60°C erhitzt, und dann werden 15 g Kaliumhydrogencarbonat (0,15 mol) zugegeben. Danach läßt man die Reaktionslösung auf Raumtemperatur abkühlen und filtriert über ein Faltenfilter ab. Man schüttelt das Rohprodukt mit 300 ml Essigsäureethylester und 700 ml Wasser aus, wäscht die organische Phase dreimal mit Wasser und engt sie in einem Rotationsverdampfer ein, bis das Reaktionsprodukt ausfällt. Das Reaktionsprodukt wird dann in einem Gemisch von Essigsäureethylester und n-Hexan (Volumenverhältnis 1:1) umkristallisiert und anschließend in einem Vakuumschrank 48 h unter Stickstoff bei 40°C/10 mbar getrocknet (Ausbeute: 94 %).

Charakterisierung:
- Massenspektrum: Molekülpeak bei 669
- Elementaranalyse:

| | | | |
|---|---|---|---|
| Theoretischer Wert(in %) | C: 61,0 | H: 3,2 | N: 2,1 |
| Ermittelter Wert(in %) | C: 61,1 | H: 3,2 | N: 2,1 |

### Beispiel 11

Herstellung von 4-(4-Amino-3-hydroxy-phenoxy)-2-(4-carboxyphenoxy) -1-trifluormethyl-3,5,6-trifluorbenzol 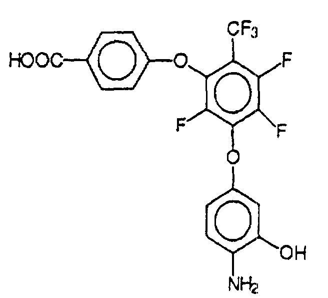 40,4 g des entsprechend Beispiel 10 hergestellten 4-(4-Nitro-3-benzyloxy-phenoxy)-2-(4-benzyloxycarbonyl-phenoxy) -1-trifluormethyl-3,5,6-trifluorbenzol (0,06 mol) werden in 500 ml eines Gemisches von Tetrahydrofuran und Essigsäureethylester (Volumenverhältnis 1:1) gelöst, und zu der Lösung werden 4 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die orangefarbene Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt in einem Vakuumschrank 48 h unter Stickstoff bei 40°C/10 mbar getrocknet (Ausbeute: 95 %).

### Charakterisierung:

- Massenspektrum: Molekülpeak bei 459
- Elementaranalyse:

| | | | |
|---|---|---|---|
| Theoretischer Wert (in %) | C: 52,3 | H: 2,4 | N: 3,0 |
| Ermittelter Wert (in %) | C: 52,3 | H: 2,4 | N: 3,0 |

## Patentansprüche

1. o-Aminophenol-carbonsäuren und o-Aminothiophenol-carbonsäuren der Struktur 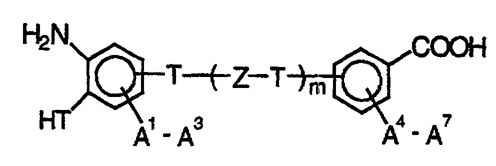 wobei folgendes gilt:
A¹ bis A⁷ sind - unabhängig voneinander - H, CH₃, OCH₃, CH₂CH₃ oder OCH₂CH₃;
T ist O oder S, und m ist 0 oder 1;
Z ist einer der folgenden Reste: 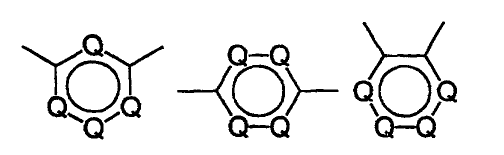 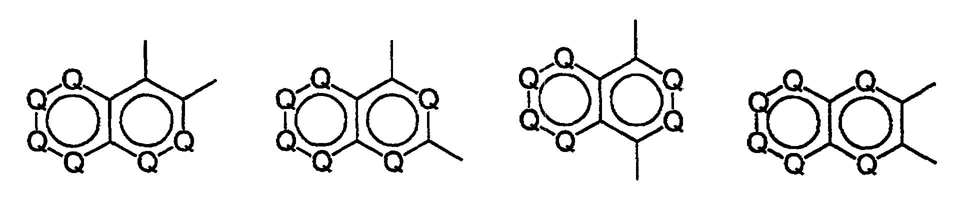 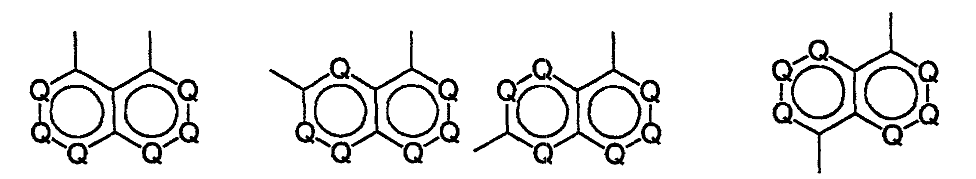 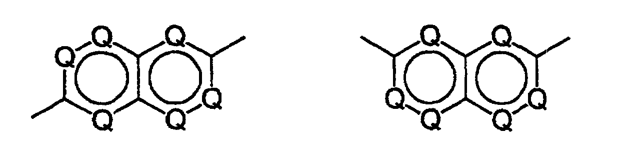 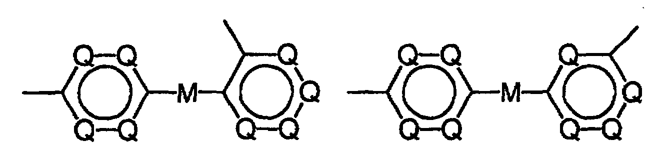 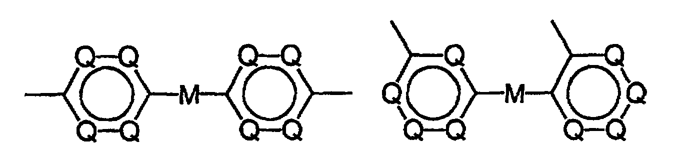 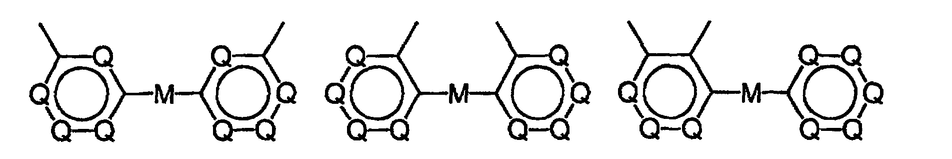 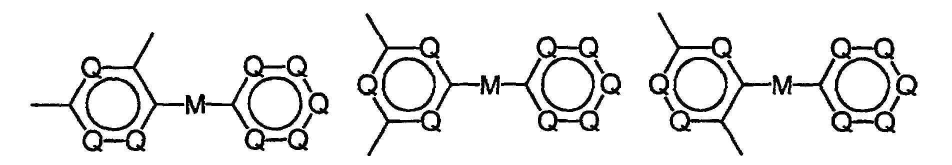 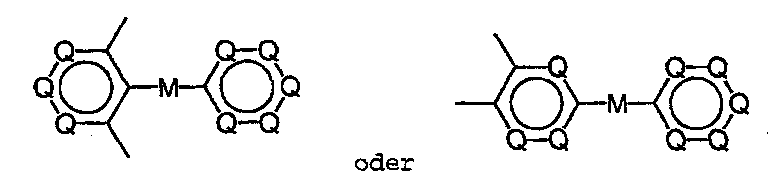
mit
Q = C-A oder N,
mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl, wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
M = eine Einfachbindung, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃) (C₆H₅), C(CH₃) (C₆F₅), C(CF₃) (C₆H₅), C(CF₃) (C₆F₅), C(C₆H₅)₂, C(C₆F₅)₂, CO, SO₂, 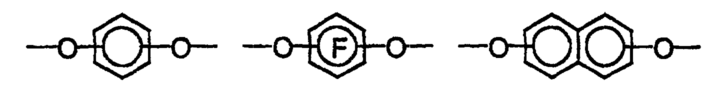 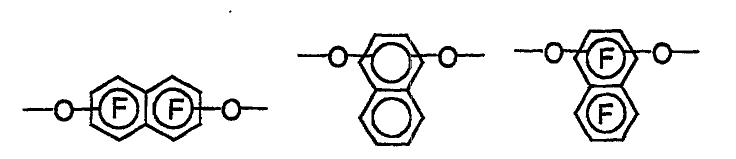 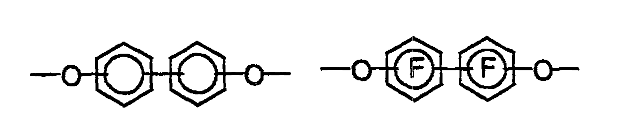 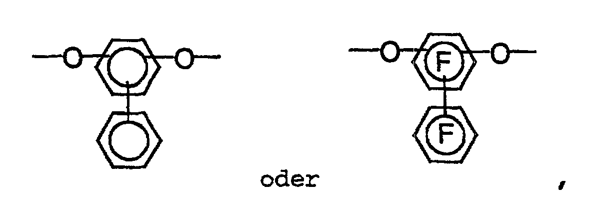
mit der Maßgabe, daß bei m = 0 keine 3-Amino-4-hydroxyphenoxygruppe in p-Stellung zur Carboxylgruppe vorliegen darf.

2. Verfahren zur Herstellung von o-Aminophenol-carbonsäuren und o-Aminothiophenol-carbonsäuren nach Anspruch 1, **dadurch gekennzeichnet, daß**
(a) eine Halogenverbindung der Struktur 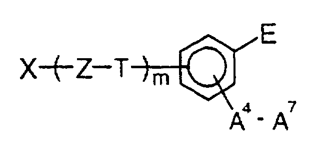 in einem Lösemittel bei einer Temperatur zwischen
-10 und 80°C
mit einem Nitrophenol bzw. Nitrothiophenol der Struktur 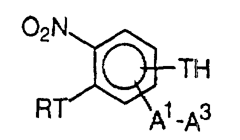 in Gegenwart mindestens der stöchiometrischen Menge einer Base oder mit einem Alkalisalz des Nitro(thio)phenols zur Reaktion gebracht wird,
wobei X ein Halogenatom ist, E die Bedeutung CN oder COOR¹ mit R¹ = Alkyl (mit 1 bis 5 C-Atomen), Phenyl oder Benzyl hat, A¹ bis A⁷, T und Z wie angegeben definiert sind und R einer der folgenden Reste ist: Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen; und
(b) die dabei gebildete Nitroverbindung zur Aminoverbindung reduziert und hydrolysiert und die Gruppe R abgespalten wird.

3. Verfahren zur Herstellung von o-Aminophenol-carbonsäuren und o-Aminothiophenol-carbonsäuren nach Anspruch 1, **dadurch gekennzeichnet, daß**
(a) eine Nitroverbindung der Struktur 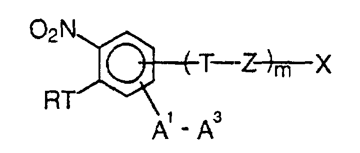 in einem Lösemittel bei einer Temperatur zwischen -10 und 80°C
mit einem Phenol bzw. Thiophenol der Struktur 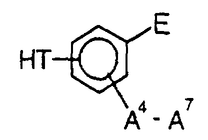 in Gegenwart mindestens der stöchiometrischen Menge einer Base oder mit einem Alkalisalz des (Thio)phenols zur Reaktion gebracht wird,
wobei X ein Halogenatom ist, E die Bedeutung CN oder COOR¹ mit R¹ = Alkyl (mit 1 bis 5 C-Atomen), Phenyl oder Benzyl hat, A¹ bis A⁷, T und Z wie angegeben definiert sind und R einer der folgenden Reste ist: Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen; und
(b) die dabei gebildete Nitroverbindung zur Aminoverbindung reduziert und hydrolysiert und die Gruppe R abgespalten wird.

4. Verfahren nach Anspruch 2 oder 3, **dadurch gekennzeichnet, daß** als Base ein Carbonat oder Hydrogencarbonat eines Alkali- oder Erdalkalimetalls eingesetzt wird.

5. Verfahren nach Anspruch 2 oder 3, **dadurch gekennzeichnet, daß** eine organische Base mit einem tertiären N-Atom eingesetzt wird.

6. Verfahren nach einem der Ansprüche 2 bis 5, **dadurch gekennzeichnet, daß** die Reduktion und die Abspaltung der Gruppe R sowie - bei E = COOR¹ - die Hydrolyse durch Wasserstoff erfolgt und mit Pd/C katalysiert wird.

## Claims

1. o-Aminophenolcarboxylic acids or o-aminothiophenolcarboxylic acids of the structure 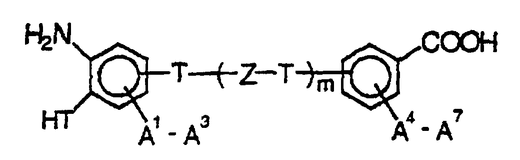 where:
A¹ to A⁷ are - independently of one another - H, CH₃, OCH₃, CH₂CH₃ or OCH₂CH₃;
T is O or S, and m is 0 or 1;
Z is one of the following radicals: 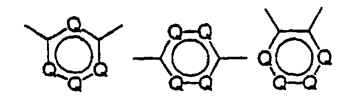 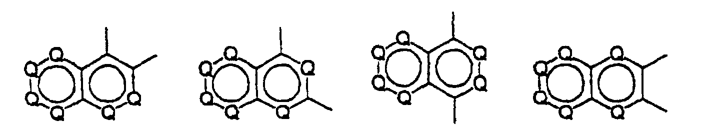 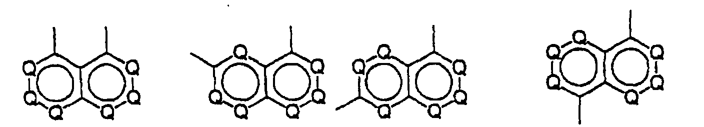 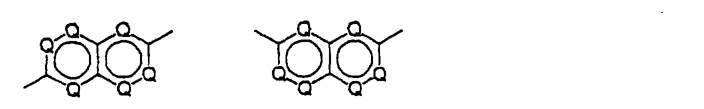 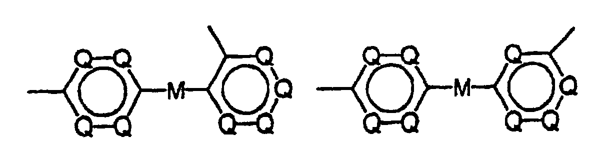 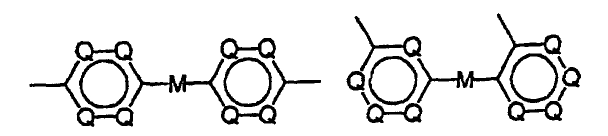 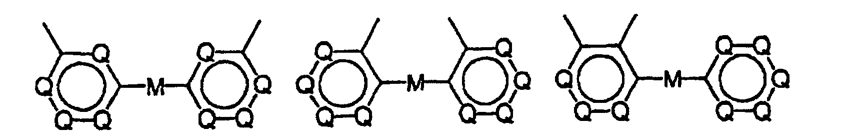 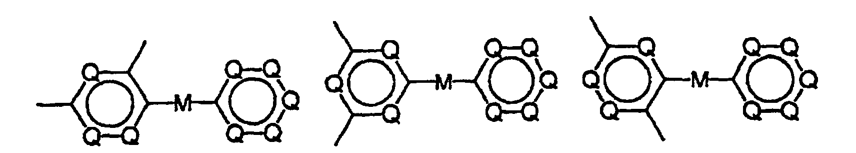 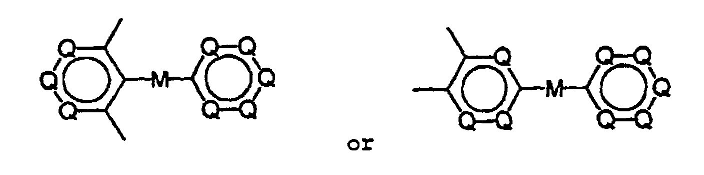
where
Q =C-A or N,
where A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ where p = 0 to 8 (linear or branched chain), OC(CH₃)₃, OC(CF₃)₃, C₆H₅, C₆F₅, OC₆H₅, OC₆F₅, cyclopentyl, perfluorocyclopentyl, cyclohexyl or perfluorocyclohexyl,
where, in the isolated aromatic rings, a maximum of 3 N atoms may be present per ring and only 2 N atoms may be adjacent, and, in the fused ring systems, a maximum of 2 N atoms may be present per ring
M = a single bond, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃) (C₆H₅), C(CH₃) (C₆F₅), C(CF₃) (C₆H₅), C(CF₃) (C₆F₅), C(C₆H₅)₂, C(C₆F₅)₂, CO, SO₂, 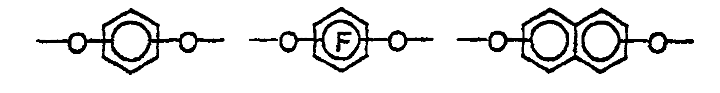 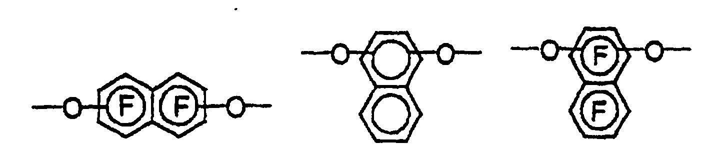 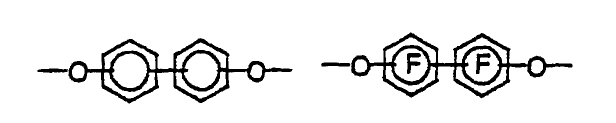 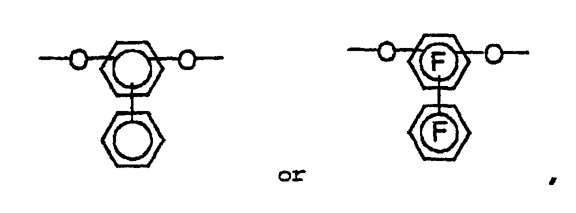 with the proviso that, when m = 0, a 3-amino-4-hydroxyphenoxy group cannot be in the p-position to the carboxyl group.

2. Process for the preparation of o-aminophenolcarboxylic acids or o-aminothiophenolcarboxylic acids according to Claim 1, **characterized in that**
(a) a halogen compound of the structure 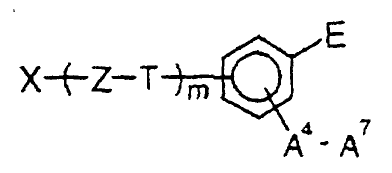 is reacted with a nitrophenol or nitrothiophenol of the structure 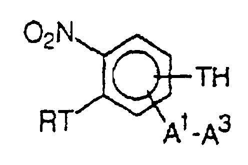 in the presence of at least the stoichiometric amount of a base, or with an alkali metal salt of the nitro(thio)phenol, in a solvent at a temperature between -10 and 80°C,
where X is a halogen atom, E is CN or COOR¹, where R¹ = alkyl (having 1 to 5 carbon atoms), phenyl or benzyl, A¹ to A⁷, T and Z are as defined above, and R is one of the following radicals: alkyl, alkoxyalkyl, alkenyl, alkoxyalkenyl, alkynyl or alkoxyalkynyl, each having a maximum of 6 carbon atoms, phenyl, phenacyl, benzyl, benzylalkyl, benzylalkenyl, benzyloxyalkyl, benzyloxyalkenyl, benzylalkoxyalkyl or benzylalkoxyalkenyl, each having a maximum of 4 aliphatic carbon atoms; and
(b) the resultant nitro compound is reduced to the amino compound, the latter is hydrolyzed, and the group R is removed.

3. Process for the preparation of o-aminophenolcarboxylic acids or o-aminothiophenolcarboxylic acids-according to Claim 1, **characterized in that**
(a) a nitro compound of the structure 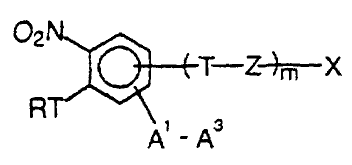 is reacted with a phenol or thiophenol of the structure 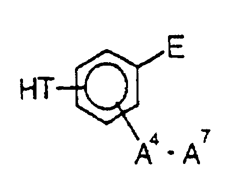 in the presence of at least the stoichiometric amount of a base, or with an alkali metal salt of the (thio)phenol, in a solvent at a temperature between -10 and 80°C,
where X is a halogen atom, E is CN or COOR¹, where R¹ = alkyl (having 1 to 5 carbon atoms), phenyl or benzyl, A¹ to A⁷, T and Z are as defined above, and R is one of the radicals : alkyl, alkoxyalkyl, alkenyl, alkoxyalkenyl, alkynyl or alkoxyalkynyl, each having a maximum of 6 carbon atoms, phenyl, phenacyl, benzyl, benzylalkyl, benzylalkenyl, benzyloxyalkyl, benzyloxyalkenyl, benzylalkoxyalkyl or benzylalkoxyalkenyl, each having a maximum of 4 aliphatic carbon atoms; and
(b) the resultant nitro compound is reduced to the amino compound, the latter is hydrolyzed, and the group R is removed.

4. Process according to Claim 2 or 3, **characterized in that** the base used is a carbonate or hydrogencarbonate of an alkali metal or alkaline earth metal.

5. Process according to Claim 2 or 3, wherein an organic base containing a tertiary N atom is used.

6. Process according to any one of Claims 2 to 5, **characterized in that** the reduction and the removal of the group R and - if E = COOR¹ - the hydrolyses are carried out by means of hydrogen and catalysed by Pd/C.

## Revendications

1. Acides o-aminophénol carboxyliques et acides o-aminothiophénol carboxyliques de formule : 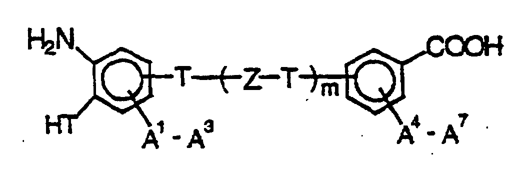 dans laquelle :
A¹ à A⁷ sont indépendamment les uns des autres H, CH₃, OCH₃, CH₂CH₃ ou OCH₂CH₃,
T est O ou S et m est 0 ou 1 ;
Z est l'un des radicaux suivants : 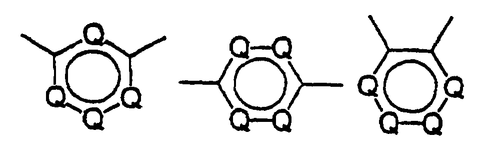 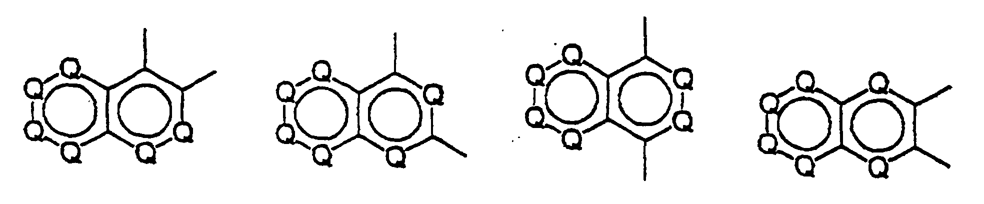 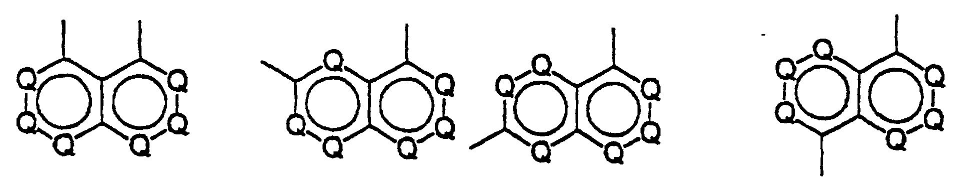 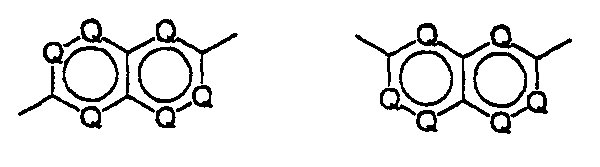 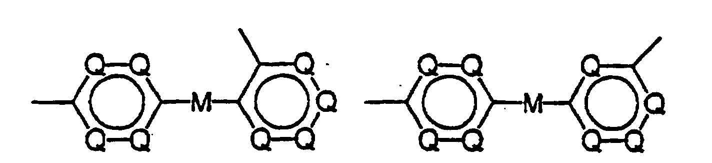 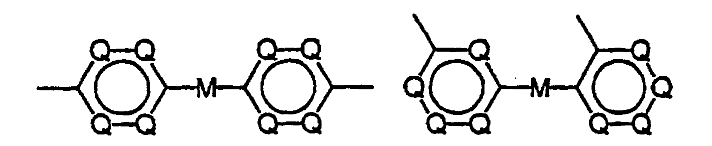 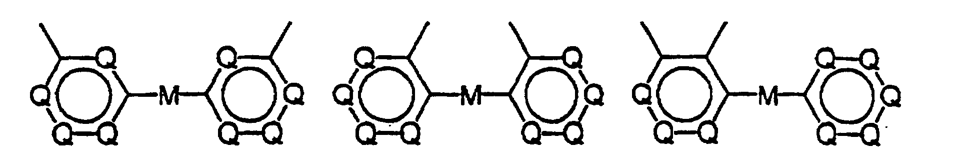 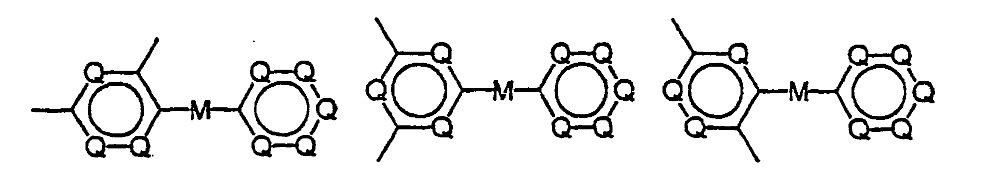 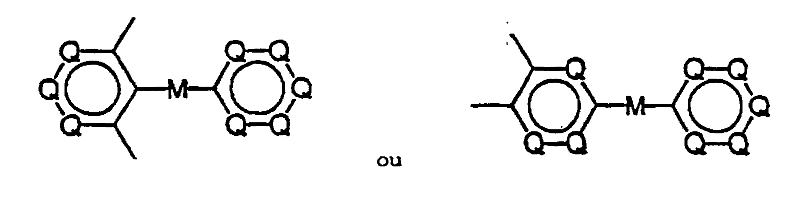
Q = C-A ou N,
avec A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ avec p = 0 à 8 (chaîne linéaire ou ramifiée),
OC(CH₃)₃, OC(CF₃)₃, C₆H₅, C₆F₅, OC₆H₅, OC₆F₅, cyclopentyle, perfluorocyclopentyle, cyclohexyle ou perfluorocyclohexyle, et il peut y avoir dans les cycles aromatiques isolés au maximum 3 atomes d'azote par cycle et seulement 2 atomes d'azote peuvent être voisins et il peut y avoir dans les systèmes cycliques condensés au maximum 2 atomes d'azote par cycle,
M = simple liaison, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃)(C₆F₅), C(C₆H₅)₂, C(C₆F₅)₂, CO, SO₂, 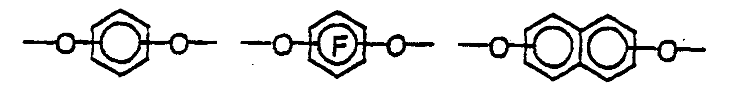 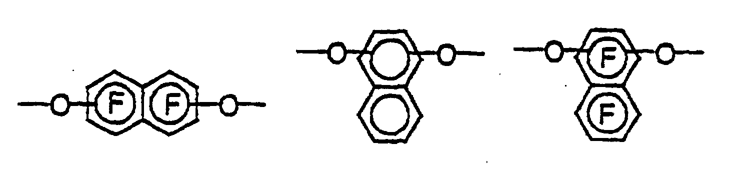 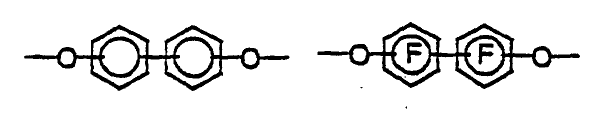 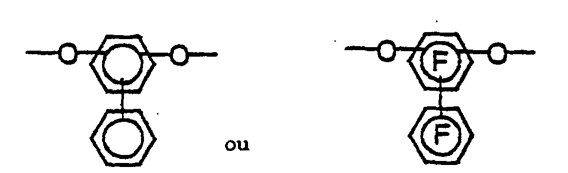
avec la condition que si m = 0 il ne doit pas y avoir de groupe 3-amino-4-hydroxy-phénoxy en position para du groupe carboxyle.

2. Procédé de préparation d'acides o-aminophénol carboxyliques et d'acides o-aminothiophénol carboxyliques suivant la revendication 1, **caractérisé en ce qu'**il consiste à mettre
(a) un composé halogéné de formule 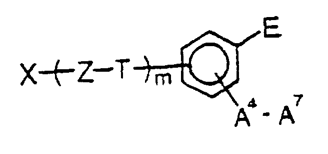 à réagir dans un solvant à une température comprise entre -10 et 80°C sur un nitrophénol ou sur un nitrothiophénol de formule 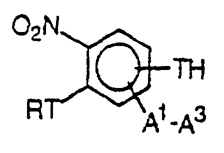 en présence d'au moins la quantité stoechiométrique d'une base ou sur un sel de métal acalin du nitro(thio)phénol,
X étant un atome d'halogène, E ayant la signification CN ou COOR¹ avec R¹ = alcoyle (ayant de 1 à 5 atomes de carbone), phényle ou benzyle et A¹ à A⁷, T et Z étant définis comme indiqué et R est l'un des radicaux suivants : alcoyle, alcoxy-alcoyle, alcènyle, alcoxyalcènyle, alcinyle ou alcoxyalcènyle ayant chacun au maximum 6 atomes de C, phényle, phénacyle, benzyle et benzylalcoyle, benzylalcènyle, benzyloxyalcyle, benzyloxyalcènyle, benzylalcoxyalcoyle ou benzylalcoxyalcènyle ayant chacun au maximum 4 atomes de C aliphatiques ; et
(b) à réduire et à hydrolyser le composé nitro ainsi formé en un composé amino et à éliminer le groupe R.

3. Procédé de préparation d'acides o-aminophénol carboxyliques et d'acides o-aminothiophénol carboxyliques suivant la revendication 1, **caractérisé en ce qu'**il consiste
(a) à mettre un composé nitro de formule 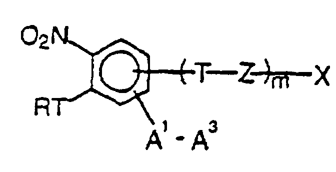 à réagir dans un solvant à une température comprise entre -10 et 80°C sur un phénol ou sur un thiophénol de formule 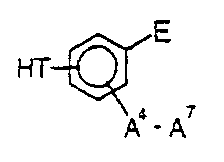 en présence d'au moins la quantité stoechiométrique d'une base ou sur un sel de métal acalin du (thio)phénol
X étant un atome d'halogène, E ayant la signification CN ou COOR¹ avec R¹ = alcoyle (ayant de 1 à 5 atomes de carbone), phényle ou benzyle et A¹ à A⁷, T et Z étant définis comme indiqué et R est l'un des radicaux suivants : : alcoyle, alcoxy-alcoyle, alcènyle, alcoxyalcènyle, alcinyle ou alcoxyalcènyle ayant chacun au maximum 6 atomes de C, phényle, phénacyle, benzyle et benzylalcoyle, benzylalcènyle, benzyloxyalcyle, benzyloxyalcènyle, benzylalcoxyalcoyle ou benzylalcoxyalcènyle, ayant chacun au maximum 4 atomes de C aliphatiques ; et
(b) à réduire et à hydrolyser le composé nitro ainsi formé en le composé amino et à éliminer le groupe R.

4. Procédé suivant la revendication 2 ou 3, **caractérisé en ce qu'**il consiste à utiliser comme basé un carbonate ou un hydrogénocarbonate d'un métal alcalin ou alcalinoterreux.

5. Procédé suivant la revendication 2 ou 3, **caractérisé en ce qu'**il consiste à utiliser une base organique ayant un atome d'azote tertiaire.

6. Procédé suivant l'une des revendications 2 à 5, **caractérisé en ce qu'**il consiste à effectuer la réduction et l'élimination du groupe R, ainsi que dans le cas où E = COOR¹ l'hydrolyse, par de l'hydrogène et à catalyser par Pd/C.
